(19) Europäisches Patentamt
European Patent Office
Office européen des brevets

(11) **EP 3 295 791 B1**

(12) **EUROPÄISCHE PATENTSCHRIFT**

(45) Veröffentlichungstag und Bekanntmachung des Hinweises auf die Patenterteilung:
**30.10.2019 Patentblatt 2019/44**

(51) Int Cl.:
*A01M 21/04* *(2006.01)*    *A61L 2/08* *(2006.01)*
*A61L 2/03* *(2006.01)*

(21) Anmeldenummer: **16188811.0**

(22) Anmeldetag: **14.09.2016**

(54) **VERFAHREN UND VORRICHTUNG ZUR BEHANDLUNG VON BEHANDLUNGSGUT**

METHOD AND DEVICE FOR TREATING TREATED ITEMS

PROCÉDÉ ET DISPOSITIF DESTINES AU TRAITEMENT DE PRODUIT À TRAITER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.03.2018 Patentblatt 2018/12**

(73) Patentinhaber: **Eberhard Bau AG**
**8302 Kloten (CH)**

(72) Erfinder:
• **VAN DER HAEGEN, Patric**
**4142 Münchenstein (CH)**
• **ALBERT, Rainer**
**4051 Basel (CH)**

(74) Vertreter: **Strässle, Simon**
**Isler & Pedrazzini AG**
**Giesshübelstrasse 45**
**Postfach 1772**
**8027 Zürich (CH)**

(56) Entgegenhaltungen:
WO-A1-95/10943    WO-A1-99/62566
CA-C- 1 298 952    DE-A1- 2 526 196
US-A- 4 670 634    US-A- 5 664 911

• DATABASE WPI Week 200737 Thomson Scientific, London, GB; AN 2007-393846 XP002767264, -& NL 1 028 075 C2 (POTVEER BV) 25. Juli 2006 (2006-07-25)

**Beschreibung**

TECHNISCHES GEBIET

Die vorliegende Erfindung betrifft ein Verfahren zur Behandlung von Behandlungsgut, insbesondere von Aushubmaterial, welches mit unerwünschten keim- und/oder fortpflanzungsfähigen pflanzlichen Beimengungen kontaminiert ist. Des Weiteren betrifft die vorliegende Erfindung eine Behandlungsvorrichtung zur Behandlung von solchem Behandlungsgut.

STAND DER TECHNIK

Die stetig wachsende Menge an mit invasiven Pflanzen wie Neophyten belastetem Aushubmaterial belastet Umwelt und Wirtschaft. Insbesondere die Verbreitung von invasiven Neophyten ist ungewollt, gesetzlich verboten und birgt grosses ökologisches und ökonomisches Schadenspotential. Neophyten belasten durch ihre aggressive Ausbreitung die heimische Pflanzenwelt und führen zu einer Verringerung der natürlichen pflanzlichen Vielfalt. Ökonomisch gesehen greifen sie Bauwerke und Infrastrukturbauten wie z.B. Mauerwerk, Strassen und Uferböschungen an (z.B. Japanischer Knöterich) und erzeugen dadurch enorme Kosten bei der Beseitigung der Schäden. Manche von ihnen sind giftig (z.B. Schmalblättriges Greiskraut), rufen nach Kontakt heftige Hautreaktionen hervor (z.B. Riesenbärenklau) oder führen zu allergischen Reaktionen (z.B. Ambrosia).

Zu bekämpfende invasive Neophyten sind länderspezifisch in sogenannten Schwarzen Listen aufgeführt. Auf diesen Listen sind bspw. Neophyten geführt, bei denen nach aktuellem Kenntnisstands ein hohes Ausbreitungspotenzial gegeben oder zu erwarten ist. Zudem ist bei diesen Neophyten der Schaden in den Bereichen Biodiversität, Gesundheit und/oder Ökonomie erwiesen und hoch. Vorkommen und Ausbreitung dieser Arten müssen verhindert werden.

Aktuelle Methoden zur Verhinderung der weiteren Verbreitung von invasiven Neophyten durch Aushub-material sind bspw. ein sicherer und abgedeckter Transport und Entsorgung in einer dafür geeigneten Deponie mit bis zu 5 m dicker Überdeckung.

Die Entsorgung von Grüngut kann in einer Kehrichtverbrennungsanlage oder durch thermophile Kompostierung erfolgen.

Eine weitere Methode zur Verhinderung der Verbreitung ist die Verwendung von Herbiziden wie Glyphosat. Herbizide könne jedoch insbesondere an Fliessgewässern aus ökologischen und umweltrelevanten Gründen nicht uneingeschränkt eingesetzt werden.

Lokale Wärmebehandlungen mit Heissdampf bei etwa 200 °C und ca. 15 Minuten werden angewendet, erfordern jedoch einen hohen Energiebedarf. Infrarotstrahlung kommt oberflächlich zum Einsatz, hat jedoch keine Tiefenwirkung.

Mechanische Methoden wie mehrmaliges Mähen oder Ausreissen können maximal eine lokale Stabilisierung der Ausbreitung invasiver Neophyten bewirken, eine Vernichtung ist schon wegen der Grösse der bereits befallenen Flächen heute nicht mehr möglich.

Mehrjähriges Abdecken befallener Flächen mit Folie führt u.a. beim Japanischen Knöterich ebenfalls maximal zu einem eng begrenzten lokalen Erfolg. Ein ähnliches Resultat gelingt durch mehrmonatiges Überfluten, falls möglich, von befallenen Flächen.

Der Einsatz ionisierender Strahlung wie u.a. Gamma-, Röntgen-, Elektronen- oder UV-Strahlung wäre möglich, erfordert aber extrem hohe Strahlendosen. Da nur ein kleiner Teil des anfallenden Materials aus invasiven Neophyten besteht sind diese Bekämpfungsmethoden unwirtschaftlich.

Aus der DE 10 2006 022 611 A1 ist ein nicht-thermisches Verfahren bekannt, gemäss dem biologisch belastetes Schüttgut auf einem Förderband zwischen zwei Elektroden Feldstärken von 0.5 kV/cm bis 10 kV/cm bei Pulsdauern (Pulshalbwertszeiten) von einigen Mikrosekunden bis einigen 10 Mikrosekunden ausgesetzt ist.

Die US 2015/0020447 A1 lehrt ein Verfahren zur Verwendung von Mikrowellen im Bereich von 500 MHz bis 1 GHz zur Zerstörung von isolierten Pflanzen. Der Einsatz von Mikrowellen (GHz-Bereich) ist jedoch für grosse Volumenströme mit entsprechendem Querschnitt ungeeignet, da die Eindringtiefe bei Mikrowellen auf einige Millimeter begrenzt ist.

Durch CA 1,298,952 lehrt ein thermisches Verfahren zur Behandlung von Bodenabdeckmaterial bekannt, gemäss dem insbesondere mit Fliegen, Pilzen und Milben verunreinigtes Pilzbrutabdeckmaterial dekontaminiert werden kann, wobei kontinuierliche, kleinere Materialströme in einem Hochfrequenzfeld von 27.12 MHz behandelt werden (maximal 20 kW HF-Leistung), sodass die Temperatur des gesamten Materials mit Verunreinigungen während 2 Minuten auf 90 °C bis 100 °C steigt. Dieses Verfahren ist explizit für Kleinst- und Kleinmengen im Kilogrammmassstab beschrieben und energieintensiv. Die WO 99/62566 A1 offenbart ein Verfahren und eine Vorrichtung zur Dekontaminierung von medizinischen Abfällen mittels Radiofrequenzbestrahlung. Die NL 1 028 075 C2 offenbart eine Vorrichtung und ein Verfahren zur Desinfektion von Anzuchterde mittels Bestrahlung im Radiofrequenzbereich.

DARSTELLUNG DER ERFINDUNG

Es ist daher eine Aufgabe der vorliegenden Erfindung, ein verbessertes Verfahren zur Eliminierung der Keim- und/oder Fortpflanzungsfähigkeit keim- bzw. fortpflanzungsfähiger Beimengungen in Behandlungsgut anzugeben.

Diese Aufgabe wird durch das Verfahren mit den Merkmalen des Anspruchs 1 gelöst. Demgemäss wird ein Verfahren zur thermischen Eliminierung der Keim- und/oder Fortpflanzungsfähigkeit von keim- und/oder fortpflanzungsfähigen pflanzlichen Beimengungen in Behandlungsgut in Form von Aushubmaterial vorgeschlagen, welches Verfahren folgende Schritte umfasst:

a) Erzeugen eines Radiofrequenzsignals mittels einer Generatoreinheit;
b) Erzeugung eines elektrischen Hochfrequenzfeldes mit dem Radiofrequenzsignal mittels einer Elektrodenanordnung;
c) Bestrahlung des Behandlungsgutes mit dem elektrischen Hochfrequenzfeld, sodass die Beimengungen auf eine maximale Temperatur von 50 °C bis 80 °C, vorzugsweise von 65 °C bis 75 °C, vorzugsweise 70°C erwärmt werden.

Vorzugsweise liegt diese maximale Temperatur also zwischen 50 °C und 80 °C, insbesondere bei über 60 °C, besonders bevorzugt zwischen 65 °C und 75 °C. Eine tiefere Temperatur kann die Keim- und/oder Fortpflanzungsfähigkeit nicht zuverlässig eliminieren, eine höhere Temperatur ist energetisch ungewünscht, da der Energieaufwand zu gross ist.

Die vorliegende Erfindung betrifft also ein thermisches Verfahren zur Eliminierung der Keim- bzw. Fortpflanzungsfähigkeit, wobei die Beimengungen insbesondere Sämlingen, Rhizomen, Wurzelwerk und/oder pflanzlicher Zellverbände bspw. von invasiven Neophyten sind oder enthalten.

Gemäss Erfindung wird Behandlungsgut mit Beimengungen, insbesondere mit invasiven Neophyten belastetes Aushubmaterial, einem hochfrequenten elektrischen Feld ausgesetzt, sodass die wasserhaltigen Beimengungen sich gezielt und schnell auf die besagte maximale Temperatur erhitzen, womit insbesondere einerseits die Keim- wie auch Fortpflanzungsfähigkeit von Sämlingen gestoppt und andererseits auch die Zellstrukturen von Rhizomen, Wurzelwerk und Grüngut irreversibel zerstört werden. Eine weitere Verbreitung ist hiermit gestoppt. Ein kontinuierliches Verfahren wird bevorzugt, womit ein kontinuierliches elektrisches Wechselfeld bevorzugt ist, zumindest solange Behandlungsgut durch die Elektrodenanordnung geführt wird.

Das Einwirken hochfrequenter elektrischer Felder auf nichtleitende Materialien ist unter dem Begriff *"Dielektrische oder Kapazitive Erwärmung"* bekannt. Bei dielektrischer oder kapazitiver Erwärmung entsteht die Wärme im nichtleitenden dielektrischen Material selbst. Die erforderliche Energie wird elektromagnetisch bei sehr hoher Frequenz (im MHz oder GHz Bereich) mittels Funkwellen übertragen. Durch das hochfrequente Wechselfeld werden speziell im Mikrowellenbereich die Moleküle mit Dipolcharakter (wie z.B. Wasser), die im Nichtleiter vorhanden sind, zu Schwingungen angeregt. Im bevorzugten Frequenzbereich von 3.5 MHz bis 100 MHz spielt dieser Dipoleffekt des Wasser eine untergeordnete Rolle, hier ist der Effekt im Wesentlichen auf die durch die gelösten Ionen erzeugte Leitfähigkeit im Wasseranteil des Materials zurückzuführen. Beide Effekte sind bedingt durch den "relativen dielektrischen Verlustfaktor $\varepsilon$''", welcher im bevorzugten Frequenzbereich von 3.5 MHz bis 100 MHz mit abnehmender Frequenz stark steigt (Komarov, V.; Wang, S.; Tang, J. Permittivity and measurements, in Encyclopedia of RF and Microwave Engineering; Chang, K., Ed.; John Wiley and Sons, Inc.: New York, NY, USA, 2005; pp. 3693-3711). Beide Effekte führen zum selben Ergebnis, die thermische Energie des zu behandelten Materials erhöht sich, die Temperatur des Stoffes wird grösser.

Da die Ionenkonzentration in Wurzeln, Rhizomen und Grüngut höher ist als im umgebenden feuchten Erdmaterial tritt dieser Erwärmungseffekt in diesen Bestandteilen des zu behandelten Materials bevorzugt auf.

Gegenüber den etablierten Methoden bietet die dielektrische Erwärmung mit Radiowellen im Kurzwellenbereich die Vorteile des direkten Energieeintrages und somit die gezielte Erwärmung von z.B. wasserhaltigen und ionenhaltigen Materialbestandteilen, worunter die pflanzlichen Beimengungen fallen. Das restliche Material des Behandlungsguts enthält in der Regel im Wasseranteil eine geringere Ionenkonzentration, womit sich dieses auch weniger stark aufheizt. Die Energie wird also gezielt in den Beimengungen platziert.

Als elektrisches Hochfrequenzfeld wird ein Feld mit einer Frequenz von 3.5 MHz bis 100 MHz genutzt. Besonders bevorzugt ist eine Frequenz von 10 MHz bis 20 MHz, insbesondere von 13.56 MHz. Diese Frequenzen eignen sich gut für Elektroden mit jeweils einer Elektrodenfläche von 1 m² bis 200 m², insbesondere von bis zu 30 m², und einem Abstand zwischen den Elektroden von 10 cm bis 100 cm oder mehr, insbesondere 20 cm bis 100 cm, besonders bevorzugt 30 cm bis 70 cm, womit zwischen den Elektroden ausreichend Platz für Materialmengen im Tonnenmassstab oder für einen Materialstrom mit beträchtlichem Querschnitt geboten ist. Hierbei kann die Spannung zwischen den Elektroden bspw. 15 kV betragen. Gleichzeitig ist mit diesen Frequenzen in einer grosstechnischen Anlage eine ausreichende Eindringtiefe in das Material oder in den Materialstrom mit einer Stärke von bis zu 1 m gewährleistet, womit die Beimengungen gezielt und durchgehend inaktiviert werden.

Gemäss einer Weiterbildung des Verfahrens beträgt eine Verweildauer des Behandlungsguts im Hochfrequenzfeld jeweils 10 Sekunden bis 600 Sekunden, insbesondere 120 Sekunden bis 300 Sekunden, besonders bevorzugt 180 Sekunden pro Volumenelement. Die Verweilzeit wird im Zusammenspiel mit der Materialmenge, mit dem Querschnitt des Massenstroms, der eingestrahlten Energiedichte, des Wassergehalts des Behandlungsguts und der gewünschten maximalen Temperatur bestimmt.

Gemäss einer Weiterbildung des Verfahrens wird das Behandlungsguts geschichtet mit einer Materialhöhe von 10 cm bis 100 cm oder mehr, insbesondere 20 cm bis 100 cm, besonders bevorzugt 30 cm bis 70 cm, dem elektrischen Hochfrequenzfeld ausgesetzt. Die Materialhöhe bemisst sich vorzugsweise in Richtung des Abstandes der zugeordneten Elektrodenpaare. Hierbei kann das Material durch mindestens eine zumindest zeitweise stationäre Elektrodenanordnung geführt werden.

Die Elektrodenanordnung kann auch aus mindestens einer mobilen Elektrode, vorzugsweise einer Bodenelektrode wie einer Sonde, Lanze oder Pflugschar bestehen und zum, in oder durch das Behandlungsgut geführt werden. Vorzugsweise sind in diesem Beispiel dann alle Elektroden der Elektrodenanordnung mobile Elektroden.

Alternativerweise kann die Elektrodenanordnung also zumindest teilweise zum Behandlungsgut geführt und dort zumindest temporär stationiert werden, also während der Behandlung ruht. Bspw. kann mindestens eine Elektrode, vorzugweise mehrere oder alle Elektroden der Elektrodenanordnung vor Ort über dem Material mit dem Behandlungsgut angeordnet oder in das Material mit dem Behandlungsgut geführt werden. Hierzu können die Elektroden als Lanzen oder Sonden in Paarung mit weiteren Lanzen oder Sonden oder Platten oder anders geformten Gegenelektroden ausgebildet sein. Es ist auch denkbar, dass deutlich mehr als zwei Elektroden, bspw. 2 bis 20 oder mehr solcher Elektroden gleichzeitig verwendet werden. Man kann bspw. eine Schar von 12 Lanzen gleichzeitig in den Boden rammen und die Behandlung dadurch auf ein grösseres Gebiet ausdehnen, was die Effizienz steigert.

Weiter ist es auch möglich, dass die Elektrodenanordnung, zumindest teilweise über oder durch das Material geführt wird, also während der Behandlung bewegt wird. So kann zumindest eine Elektrode, vorzugweise mehrere oder alle Elektroden der Elektrodenanordnung durch oder über das Material mit dem Behandlungsgut oder das Behandlungsgut geführt werden. Dazu kann bspw. die Elektrodenanordnung als Pflugschar ausgebildet sein und mittels einer Zugmaschine über oder durch das Material mit dem Behandlungsgut oder durch das Behandlungsgut selbst gezogen werden. Es können hier auch wieder zwei oder mehr Elektroden in der Elektrodenanordnung verwendet werden.

Eine typische Dichte von Aushub ist 1.0 g/cm$^3$ bis 3.0 g/cm$^3$ und dessen Wassergehalt beträgt 5 % bis 40 %.

Gemäss einer bevorzugten Weiterbildung des Verfahrens wird das Behandlungsgut kontinuierlich durch das elektrische Hochfrequenzfeld geführt. Eine kontinuierliche Beförderung des Behandlungsguts wird vorzugsweise durch ein Förderband ermöglicht. Andere Beförderungsanlagen sind hier denkbar. Wie hierin beschrieben ist auch denkbar, dass die Elektroden zum Behandlungsgut transportiert werden, bspw. als Lanzen oder Pflugscharen auf einer Zugmaschine, bspw. einem Traktor oder einem ähnlichen Gefährt.

Gemäss einer Weiterbildung des Verfahrens wird Behandlungsgut in der Menge von 10 Tonnen bis 500 Tonnen pro Stunde, insbesondere von 100 Tonnen bis 200 Tonnen pro Stunde in einer Grossanlage behandelt. Hierzu weist die Grossanlage eine Fördervorrichtung auf. Es können dabei mehrere parallel laufende und behandelnde Förderstrassen mit jeweils separaten oder geteilten Generatoreinheiten benützt werden.

Die vorliegende Erfindung betrifft eine Behandlungsvorrichtung zur erfindungsgemässen Behandlung von Behandlungsgut in Form von Aushubmaterial, wobei das Behandlungsgut keim- und/oder fortpflanzungsfähige Beimengungen aufweist und die Behandlung die thermische Eliminierung der Keim- und/oder Fortpflanzungsfähigkeit der keim- und/oder fortpflanzungsfähigen Beimengungen umfasst, wobei die Behandlungsvorrichtung umfasst:

mindestens eine Generatoreinheit zur Bereitstellung eines Radiofrequenzsignals,
mindestens eine Elektrodenanordnung umfassend mindestens eine erste Elektrode und mindestens eine zweite Elektrode zur Bereitstellung eines elektrischen Hochfrequenzfeldes aufgrund des Radiofrequenzsignales;
wobei die Behandlungsvorrichtung eingerichtet ist, die pflanzlichen Beimengungen auf eine maximale Temperatur von 50 °C bis 80 °C, vorzugsweise von 65 °C bis 75 °C zu erwärmen.

In einer Weiterbildung umfasst die Behandlungsvorrichtung mindestens eine Fördervorrichtung zur Beförderung von Behandlungsgut durch das elektrische Hochfrequenzfeld.

Die mindestens einen ersten und zweiten Elektroden sind, wie oben beschreiben, als mobile Elektroden in Form von Pflugscharen ausgebildet, welche temporär zum, in oder durch das Behandlungsgut geführt und dort stationiert oder bewegt werden. Die Elektroden sind als Pflugscharen ausgebildet und werden zum Pflügen unter gleichzeitiger erfindungsgemässer Behandlung verwendet.

In einer Weiterbildung ist die Behandlungsvorrichtung eingerichtet, eine Verweildauer des Behandlungsguts im elektrischen Hochfrequenzfeld von 10 Sekunden bis 600 Sekunden, insbesondere von 120 Sekunden bis 300 Sekunden im elektrischen Hochfrequenzfeld zu realisieren.

Gemäss einer Weiterbildung ist vorgesehen, dass die Behandlungsvorrichtung eine Wärmerückgewinnungsvorrichtung umfasst, welche eingerichtet ist,

a) behandeltes Behandlungsgut und/oder die Generatoreinheit mit einem Austauschfluid in Wärmekontakt zu bringen, sodass das Austauschfluid Wärme aufnimmt, und danach

b) das Austauschfluid mit der aufgenommenen Wärme mit zur Behandlung vorgesehenem Behandlungsgut zwecks Vorwärmung in Kontakt zu bringen.

Das Austauschfluid kann insbesondere Luft oder ein Austauschgas sein.

Gemäss einer Weiterbildung ist die Fördervorrichtung ausgebildet, dass das Behandlungsgut kontinuierlich durch das elektrische Hochfrequenzfeld zu führen, wobei dann ein kontinuierliches Hochfrequenzfeld besonders vorteilhaft ist, und wobei die Fördervorrichtung vorzugsweise ein einzelnes oder aber eine Vielzahl von parallelen und/oder sich aneinander anschliessenden Förderbändern umfasst. Das kontinuierlich betreibbare Förderband kann einzelne Haufen von Behandlungsgut kontinuierlich durch das Hochfrequenzfeld führen. Es kann auch eine Vorrichtung vorgesehen sein, welche einen kontinuierlichen Massenstrom mit einem vorbestimmten Querschnitt auf dem Förderband sicherstellt, wobei das zu behandelnde Behandlungsgut in einer Aufgabestation gesammelt wird und von dort kontinuierlich auf das Förderband gelangt.

Gemäss einer Weiterbildung weist die Fördervorrichtung eine Vielzahl von parallelen Förderstrassen auf, wobei jede Förderstrasse vorzugsweise mindestens eine oder zwei Elektrodenanordnung aufweist, wobei die Behandlungsvorrichtung vorzugsweise ausgelegt ist, um Behandlungsgut in der Menge von bspw. 10 Tonnen bis 500 Tonnen pro Stunde, insbesondere von 100 Tonnen bis 200 Tonnen pro Stunde zu behandeln. Eine Förderstrasse kann durch ein einzelnes Förderband oder durch mehrere, in Förderrichtung aneinander anschliessende Förderbänder bereitgestellt werden.

Das elektrische Hochfrequenzfeld weist eine Frequenz von 3.5 MHz bis 100 MHz, insbesondere von 10 MHz bis 20 MHz, besonders bevorzugt 13.56 MHz auf. Es sei hier auf die oben genannten Vorteile dieser Frequenzwahl verwiesen.

Gemäss einer Weiterbildung liegt eine Elektrodenfläche der ersten und/oder zweiten Elektrode im Bereich zwischen 1 $m^2$ und 200 $m^2$, insbesondere bei 2 $m^2$ bis 100 $m^2$, besonders bevorzugt zwischen 3 $m^2$ und 30 $m^2$. Die erste und/oder zweite Elektrode kann selbst auch eine Anordnung aus mehreren Elektroden sein, welche dann gemeinsam eine Elektrodenfläche der genannten Grösse aufweisen. Diese grossdimensionierten Elektrodenflächen erlauben es, einen gewünschten Stundendurchsatz von bis zu einigen hundert Tonnen Behandlungsgut sicherzustellen.

Gemäss einer Weiterbildung weist die eine Generatoreinheit oder weisen die mehrere Generatoreinheiten zusammen eine Ausgangsleistung 20 kW bis 3 MW auf. Eine Ausgangsspannung der Generatoreinheit kann 0.5 kV bis 15 kV betragen.

Gemäss einer Weiterbildung umfasst die Behandlungsvorrichtung eine Waschvorrichtung zur Reinigung der Transportmittel, insbesondere der Lastwagen, sodass das Transportmittel während der Behandlung des angelieferten Behandlungsguts gewaschen werden kann. Das Transportmittel ist zum Transport des Prozessguts ausgebildet und kann insbesondere ein Lastwagen, ein Güterzug, ein Lastschiff, ein Bagger, ein Radlader, eine Fördervorrichtung wie ein Förderband oder ein anderes gebräuchliches Fördermittel sein. Das Transportmittel ist hierbei ausgebildet, die kontaminierte Ladung der Behandlungsvorrichtung zu übergeben. Die Behandlungsvorrichtung führt die Behandlung durch, setzt also das Behandlungsgut nach dessen Annahme dem elektrischen Hochfrequenzfeld aus. In der Zwischenzeit, bspw. wenige Minuten bis wenige Stunden nach der Abgabe des unbehandelten Behandlungsguts an die Behandlungsvorrichtung, kann das Transportmittel an einer Waschstation der Behandlungsvorrichtung gereinigt werden. Damit werden allfällige kontaminierte Rückstände aus dem Transportmittel entfernt und das Transportmittel kann das behandelte aber auch anderes behandelte, also dekontaminiertes Behandlungsgut entgegennehmen, ohne dass die Gefahr einer erneuten Verunreinigung durch Rückstände besteht.

Die Erfindung betrifft weiter die Verwendung des erfindungsgemässen Verfahrens und/oder der erfindungsgemässen Behandlungsvorrichtung zur Behandlung von Behandlungsgut mit pflanzlichen Beimengungen, wobei diese pflanzlichen Beimengungen insbesondere Neophyten umfassen oder Neophyten sind.

Die vorliegende Erfindung betrifft also ein thermisches Verfahren zur Eliminierung der Keim- bzw. Fortpflanzungsfähigkeit insbesondere von Sämlingen, Rhizomen, Wurzelwerk und pflanzlicher Zellverbände bspw. von invasiven Neophyten, welche insbesondere durch Aushubmaterial verbreitet werden.

Gemäss der Erfindung wird Behandlungsgut mit pflanzlichen Beimengungen, bspw. mit invasiven Neophyten belastetes Behandlungsmaterial in Form von Aushubmaterial, einem hochfrequenten elektrischen Feld ausgesetzt und auf die maximale Temperatur erhitzt, welche 50 °C bis 80 °C betragen kann. Damit kann einerseits die Keim- wie auch Fortpflanzungsfähigkeit bspw. von Sämlingen gestoppt und andererseits auch die Zellstrukturen von Rhizomen, Wurzelwerk und Grüngut irreversibel zerstört werden. Eine weitere Verbreitung ist hiermit gestoppt.

Die vorliegende Erfindung stellt also ein wirtschaftliches Verfahren und auch eine Anlage bereit, um entweder bereits am Entstehungsort des mit Neophyten belasteten Behandlungsguts in Form von Aushubmaterial, eine Eliminierung der Belastung zu ermöglichen, oder an einem, vom Entstehungsort räumlich entfernt und speziell dafür geeigneten zentralen Ort, wie bspw. einer Aufbereitungsanlage, eine Eliminierung der Belastung zu ermöglichen. Das Verfahren kann für Materialströme und Mengen von bis zu mehreren 100 Tonnen Stundenleistung dimensioniert sein.

KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Fig. 1    einen schematischen Aufbau zur Erzeugung eines elektrischen Hochfrequenzfeldes mit einer Generatoreinheit;
Fig. 2    einen schematischen Aufbau einer erfindungsgemässen Behandlungsvorrichtung;
Fig. 3    eine Grossanlage mit mehreren Behandlungsvorrichtungen nach Fig. 2; und
Fig. 4    einen schematischen elektrischen Aufbau der Behandlungsvorrichtung nach Fig. 2.

BESCHREIBUNG BEVORZUGTER AUSFÜHRUNGSFORMEN

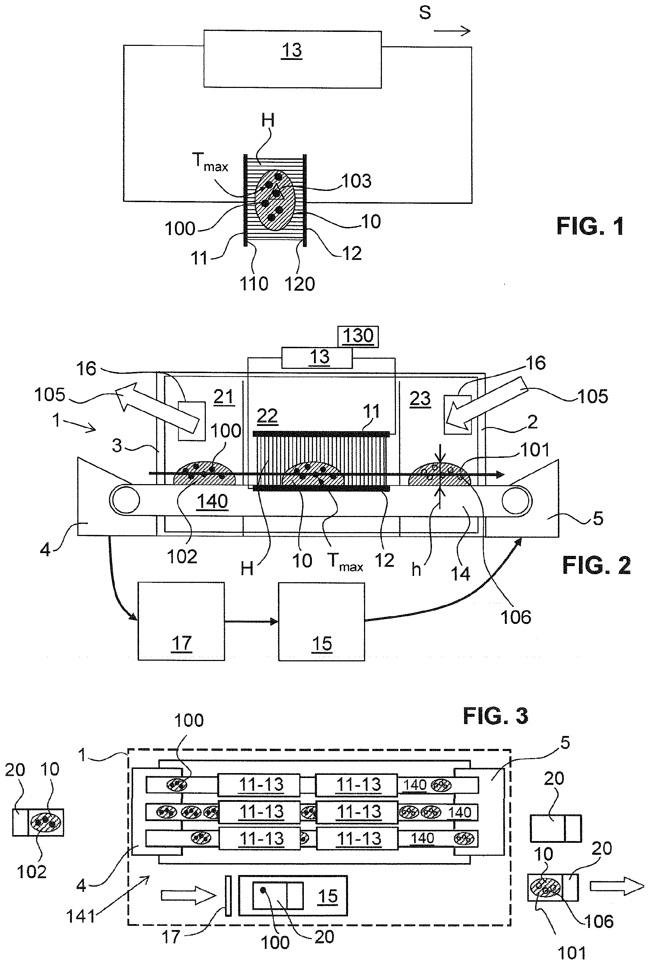

**Figur 1** zeigt das Grundprinzip der Erzeugung der elektrischen Wechselfelder und die Behandlung des Behandlungsguts 10 im Detail. **Figur 2** zeigt in schematischer Weise eine bevorzugte Ausführungsform der erfindungsgemässen Behandlungsvorrichtung 1.

Die Behandlungsvorrichtung 1 weist eine Behandlungsgutannahme 4, eine Fördervorrichtung 14 und eine Behandlungsgutabgabe 5 auf. Die Fördervorrichtung 14 führt Behandlungsgut 10 aus der Behandlungsgutannahme 4 in die Behandlungsgutabgabe 5. Dabei führt die Fördervorrichtung 14 das Behandlungsgut 10 durch ein Gehäuse 2.

Die Behandlungsgutannahme 4 ist derart ausgestaltet, dass ein Transportmittel 20 (s. Fig. 3), insbesondere ein Lastwagen, Schiff oder Zug, Bagger oder Radlader oder Dergleichen, zu behandelndes Behandlungsgut 102 mit aktiven Beimengungen 100 ebendort der Behandlungsvorrichtung 1 übergeben kann.

Die Behandlungsgutabgabe 5 ist derart ausgestaltet, dass dem vorzugsweise gereinigten Transportmittel 20 behandeltes Behandlungsgut 102, vorzugsweise aber nicht zwingenderweise das vom besagten Transportmittel 20 angelieferte, mit den inaktivierten Beimengungen 106 wieder übergeben werden kann.

Die Fördervorrichtung 14 umfasst ein Förderband 140, auf welchem das Behandlungsgut 10 durch das Gehäuse 2 transportierbar ist. Eine typische Förderbandgeschwindigkeit kann etwa 1 m/min bis 10 m/min, insbesondere 1 m/min bis 3 m/min oder 1.33 m/min, betragen. Eine typische Breite des Förderbandes 140 kann 0.5 m bis 2 m, insbesondere 1 m betragen.

Das Gehäuse 2 weist eine erste Kammer 21, eine zweite Kammer 22 und eine dritte Kammer 23 auf. Zudem ist das Gehäuse 2 mit einer Wärmeisolation 3 umgeben. Die Isolation 3 sorgt dafür, dass der Energieaufwand durch Wärmeverluste klein gehalten wird und dass sich ein vorbestimmtes Klima im Gehäuse 2 einstellt.

Das Behandlungsgut 10 wird aus der Behandlungsgutannahme 4 entnommen und mittels der Förderanlage 14 der Reihe nach durch die drei sich aneinanderschliessenden Kammern 21, 22, 23 zur Behandlungsgutabgabe 5 geführt. Die drei Kammern 21, 22, 23 bilden sozusagen einen Tunnel, durch welchen das Behandlungsgut 10 geführt wird.

Ausserhalb dieses Tunnels ist eine Generatoreinheit 13 mit der zugehörigen Kühleinheit 130 angeordnet. Die Generatoreinheit 13 kann, je nach Bedarf, eine Leistung von bspw. 20 kW bis 500 kW für eine Radiosignal S bei einer Frequenz von 3.5 MHz bis 50 MHz mit einer Ausgangsspannung von 5 kV bis 15 kV für die Speisung einer Elektrodenanordnung 11, 12 zwecks Erzeugung des Hochfrequenzfeldes H aufweisen.

In der zweiten Kammer 22 ist die Elektrodenanordnung angeordnet. Die Elektrodenanordnung umfasst eine erste Elektrode 11 mit einer zum Förderband 140 gerichteten ersten Elektrodenfläche 110 und eine zweite Elektrode 12 mit einer zum Förderband 140 gerichteten zweiten Elektrodenfläche 120 (s. auch Fig. 1). Die erste und zweite Elektrodenflächen 110, 120 sind im Wesentlichen gleich gross und weisen eine Fläche von 1 $m^2$ bis 200 $m^2$, insbesondere von 2 $m^2$ bis 100 $m^2$, besonders bevorzugt von mehr als 3 $m^2$ auf. Die erste Elektrode 11 ist oberhalb des Förderbandes 140, die zweite Elektrode 12 unterhalb des Förderbandes 140 platziert, sodass das Behandlungsgut 10 auf dem laufenden Förderband 140 zwischen den beiden Elektroden 11, 12 durchgeführt wird.

Die zweite Elektrode 12 kann hierbei direkt als Auflage für das Förderband 140 dienen und das Förderband 140 abstützen, damit ein Durchhängen des Förderbandes 140 oder der Last der geführten Behandlungsguts 10 verkleinert ist. In dieser Ausbildung steht das Förderband 140 in einem Schleifkontakt mit der zweiten Elektrode 12.

Die Elektrodenanordnung 11, 12 und die Generatoreinheit 13 sind nun derart ausgebildet, dass mittels der Generatoreinheit 13 ein elektrisches Hochfrequenzfeld H zwischen der ersten Elektrode 11 und der zweiten Elektrode 12 anlegbar ist.

Die erste Kammer 21 dient als Vorwärmkammer. Es herrscht dort eine typische Temperatur von 40 °C bis 60 °C und eine typische relative Luftfeuchtigkeit von 40 % bis 80 %. Abwärme aus der Kühlung 130 der Generatoreinheit 13 und des durch Einwirkung des Hochfrequenzfeldes H in der zweiten Kammer 22 aufgeheizten Behandlungsgutes 10 wird aus der dritten Kammer 23 über eine Wärmerückgewinnungsvorrichtung 16 zurückgewonnen und in die erste Kammer 21 zwecks Vorwärmung des zu behandelnden Behandlungsguts 102 rückgeführt. Die Wärmerückgewinnungsvorrichtung 16 kann bspw. ein Austauschfluid 105 wie etwa Luft nutzen. Dieses Austauschfluid 105 kann mit dem behandelten heissen Behandlungsgut 101 in Kontakt gebracht werden, bspw. über das Behandlungsgut 101 geströmt werden und/oder bspw. mit der Kühlung 130 der Generatoreinheit 13 in Kontakt gebracht werden, sodass sich das Fluid 105 über eine Temperatur des zu behandelnden Behandlungsguts 102 aufwärmt. Das aufgewärmte Fluid 105 kann sodann über das kalte, zu behandelnde Behandlungsgut 101 geströmt werden, um letzteres vorzuwärmen. Damit wird die durch die Behandlung mittels des Hochfrequenzfeldes H erzeugte Wärme und die in der Generatoreinheit 13 entstehende Abwärme teilweise rezykliert.

Belastetes Behandlungsgut 102, also bspw. Aushubmaterial, mit den aktiven Beimengungen 100 kann also in der Aufgabestation 4 entladen und über das Förderband 140 in die Vorwärmzone der ersten Kammer 21 transportiert werden, mit der Absicht die Prozesswärme aus dem eigentlichen Behandlungsprozess wie auch die Abwärme aus der Generatoreinheit optimal auszunutzen. Anschliessend wird das so vorgewärmte Material 10 in der mittels der Generatoreinheit 13 mit dem Hochfrequenzfeld H beaufschlagten Elektrodenanordnung 11, 12 während einer Verweilzeit von bspw. 1 Minute bis 10 Minuten zwischen den Elektroden 11, 12 durch thermische Behandlung bei $T_{max}$ von 50 °C bis 80 °C inertisiert. Durch die Einwirkung des Hochfrequenzwechselfeldes H auf das mit invasiven Neophyten belastete Aushubmaterial werden die pflanzlichen Anteile thermisch in einem Zeitraum von Minuten dermassen geschädigt, dass eine Keim- und/oder Fortpflanzungsfähigkeit nicht mehr gegeben ist.

Ein kontinuierliches Verfahren wird bevorzugt. Dazu kann das Prozessgut, also das Behandlungsgut 10, während der Verweilzeit durch das Hochfrequenzfeld H gefahren werden. Die Geschwindigkeit des Förderbandes 140 ist also entsprechend der Ausdehnung des Hochfrequenzfeldes H entlang des Förderbands 140 und der gewünschten Verweildauer im Feld H zu wählen.

Es ist auch denkbar, dass die Elektrodenanordnung mehrere sich aneinanderreihende Elektrodenpaare aufweist, welche sich direkt aneinander anschliessend oder beabstandet zueinander angeordnet sein können.

Bei diesem Prozessschritt der Behandlung des Guts 10 mit dem Hochfrequenzfeld H erwärmt sich die aktiven Beimengung 100, je nach Verweilzeit, auf 50 °C bis 80 °C, was die aktiven, d.h. keim- und/oder fortpflanzungsfähigen, Beimengungen 100 inaktiviert. Das so behandelte Behandlungsgut 101 mit den inaktivierten Beimengungen 106 wird über das Förderband 140 ausgeschleust und der Behandlungsgutabgabe 5 übergeben. Von dort kann es mittels des inzwischen gewaschenen oder anderen Transportmittels 20 (s. Fig. 3), insbesondere einem Lastwagen, abtransportiert werden.

Die Behandlungsvorrichtung 1 kann auch eine Bezahlstation 17 und eine Waschstation 15 umfassen.

An der Bezahlstation 17 kann die beanspruchte Dienstleistung direkt bezahlt werden. An der Waschstation 15, welcher integraler Bestandteil der Behandlungsvorrichtung 15 sein kann, kann das Transportmittel 20 gereinigt werden. Insbesondere kann die Ladefläche zur Aufnahme des behandelten Behandlungsguts 101 von allfällig aktiven Rückständen, welche nicht vollständig an der Aufgabestation 4 der Behandlungsvorrichtung 1 übergeben wurden, befreit werden. Dies stellt sicher, dass das behandelte Gut 101 bei der Entgegennahme von Station 5 nicht wieder kontaminiert wird.

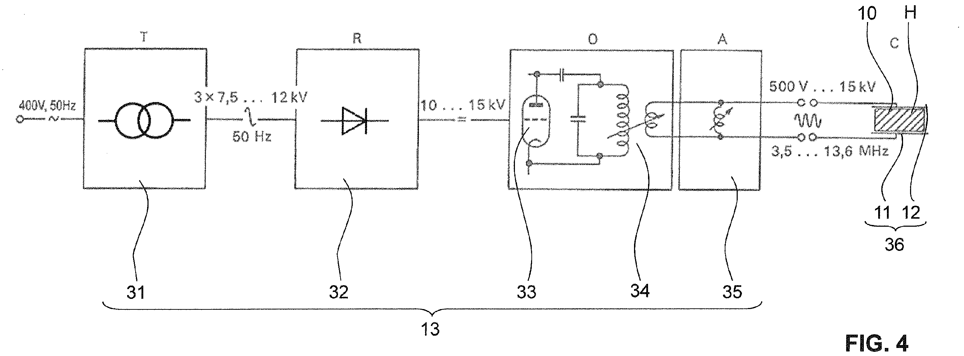

**Figur 4** zeigt in schematischer Weisen den elektrischen Aufbau der Behandlungsvorrichtung, also den dem Funktionsprinzip nach Fig. 1 zu Grunde liegenden Schaltkreises.

Die mit *T* bezeichnete Stufe zeigt den Vorgang, bei welchem die Netzspannung (400 V, 50 Hz) mittels eines Transformators 31 in Hochspannung (3 x 7.5 kV bis 12 kV, 50 Hz) umgewandelt.

Die mit *R* bezeichnete Stufe zeigt den Vorgang, bei welchem die besagte Hochspannung (3 x 7.5 kV bis 12 kV, 50 Hz) dann mittels eines Hochspannungsgleichrichters 32 in Gleichspannung (10 kV bis 15 kV) umwandelt wird.

Die mit *O* bezeichnete Stufe zeigt den Vorgang, bei welchem die besagte hohe Gleichspannung (10 kV bis 15 kV) einer im Oszillatorteil montierten Leistungstriode 33 zugeführt wird. In diesem Schwingkreis entsteht eine Hochspannung hoher Frequenzen (3.5 MHz bis 13.6 MHz).

Die mit *A* bezeichnete Stufe zeigt den Vorgang, bei welchem die hochfrequente Hochspannung über eine variable oder fixe Koppelspule 34 an die Anpassvorrichtung 35 legt, die im Prinzip aus einer variablen Induktivität oder variablen Kapazität besteht. Damit wird, durch Motorantrieb, die Impedanz 36 optimal abgestimmt, bzw. die Arbeitsspannung eingestellt.

Die mit *C* bezeichnete Stufe zeigt den Vorgang, bei welchem im parallel zur Anpassvorrichtung 35 geschalteten Arbeitskondensator 36 ein hochfrequentes elektrisches Wechselfeld H entsteht, das die Erwärmung des zwischen den Kondensatorplatten eingelegten Gutes bewirkt. Die Kondensatorplatten wirken also als Elektroden 11, 12 wo eine Spannung von 500 V bis 15 kV herrschen kann.

Anwendung findet diese Methode in der Holzindustrie wobei z.B. die Trocknungszeit oder die Verleimungszeit verkürzt wird. Eine entsprechende Literaturstelle ist z.B. von Dipl. Ing. Ch. Alt in "Chemie Ingenieur Technik; Volume 37, Issue 12, pages 1229-1234, December 1965" gegeben.

Im hochfrequenten elektrischen Wechselfeld H lassen sich nichtleitende dielektrische Materialen erwärmen (dielektrische oder kapazitive Erwärmung). Die Energieaufnahme (im Dielektrikum erzeugte Leistung) wird durch folgende Gleichung beschrieben:

$$P = 2 \pi \, \varepsilon_o \, f \, \varepsilon'' \, E^2 \, A \, h_K \, [\text{W}]$$

wobei

$f$ = Betriebsfrequenz

$\varepsilon_o$ = dielektrische Feldkonstante = $8.85 \times 10^{-12}$ [As/Vm]

$\varepsilon'$ = relative Dielektrizitätskonstante

$\varepsilon''$ = relativer dielektrischer Verlustfaktor

$E$ = elektrische Feldstärke; E = Spannung/Plattenabstand [V/m]

$A$ = Fläche der Kondensatorplatte [m$^2$]

$h_K$ = Kondensatorplattenabstand [m]

also

$$P = (2 \pi \, \varepsilon_o \, E^2) \, f \, \varepsilon'' A \, h = K_{\text{onstante}} \, f \, \varepsilon'' \, A \, h$$

Die mögliche Materialhöhe h liegt im Bereich des Kondensatorplattenabstandes $h_K$, kann also von 10 cm bis 100 cm betragen, bedingt durch die Korngrössenverteilung und den Schüttwinkel des aufgegebenen Materials.

Mit vorgegebener Materialhöhe h des Materialstromes aus Behandlungsgut 10 ist die adsorbierte Energie massgeblich vom relativen dielektrischen Verlustfaktor $\varepsilon''$, von der Frequenz f sowie von der Elektrodenfläche 110, 120 abhängig. Zur vollständigen Durchdringung des Materials 10 ist die frequenzabhängige Eindringtiefe ET der Wellen von Bedeutung.

Die Eindringtiefe ET der Wellen hängt von den elektromagnetischen Eigenschaften des Materials ab und ist gegeben durch die Gleichung:

$$ET = \frac{1}{2a} \text{ wobei für } \alpha \text{ gilt:} \qquad \alpha = \frac{2\pi}{\lambda_o} \left[ \frac{1}{2} \varepsilon' \left( \sqrt{1 + \left( \frac{\varepsilon''}{\varepsilon'} \right)^2} - 1 \right) \right]^{\frac{1}{2}}$$

Die Eindringtiefe ET der Wellen nimmt mit steigender Frequenz ab. (s. A. Von Hippel, Dielectrics and Waves, Wiley, New York, 1954).

Für Wasser z.B. ($\varepsilon'$ = 77.4 und $\varepsilon''$ = 9.2 bei 2.54 GHz) gilt demnach bei

| ISM Frequenz | Wellenlänge ($\lambda_0$) | Eindringtiefe (ET) |
|---|---|---|
| 2.54 GHz $\pm$ 50 MHz (Mikrowellen) | 12.24 cm | < 0.1 m (Wasser) |
| 2.54 GHz $\pm$ 50 MHz (Mikrowellen) | 12.24 cm | 0.01 - 0.1 m (feuchte Böden) |
| 27.12 MHz $\pm$ 0.163 MHz (Kurzwellen) | 1106.2 cm | 7 m (feuchte Böden) |
| 13.56 MHz $\pm$ 15 kHz (Kurzwellen) | 2212.4 cm | 18 m (feuchte Böden) |
| ISM = Industrial, Scientific and Medical Band | | |

Die geringere Eindringtiefe ET im Fall der Mikrowellen-Anregung führt dazu, dass die Energie zwar effizient in Wärme umgesetzt wird, die Wirkung jedoch auf den Oberflächenbereich (einige mm bis cm) beschränkt bleibt. Aus diesem Grund sind für die Behandlung grösserer Volumina Radiowellen vorteilhaft, um Eindringtiefen ET im Meter-Bereich zu realisieren.. Bevorzugte Frequenzen (3.5 MHz bis 100 MHz) im Kurzwellenbereich ergeben für den vorgegebenen Materialstrom Elektrodenflächen im Bereich von 1 m² bis 200 m², insbesondere von 4 m² oder 3 m² bis 30 m².

Die Leistung in Betracht ziehend ist im Bereich der Radiofrequenzen zwischen 3.5 bis 100 MHz der Faktor $\varepsilon''$ (relativer dielektrischer Verlustfaktor) von wesentlicher Bedeutung. Wird dieser im Mikrowellenbereich vorwiegend von den dipolaren Eigenschaften von Wasser beeinflusst, sind im bevorzugten Frequenzband zwischen 3.5 MHz bis 100 MHz die ionische Einflüsse wie bspw. die Leitfähigkeit entscheidend. Der Einfluss steigt exponentiell an mit abnehmender Frequenz (s. bspw.: J. Tang, H. Feng, and M. Lau, Microwave heating in food processing, in X. Young and J. Tang, eds., Advances in Bioprocessing Engineering, World Scientific, 2002), sodass für den Energieeintrag niedrigere Frequenzen von Vorteil sind.

Es gilt:

$$\varepsilon'' = \varepsilon_d'' + \varepsilon_\sigma'' = \varepsilon_d'' + \frac{\sigma}{\varepsilon_o \omega}$$

wobei "d" und "$\sigma$" für die Beiträge aus dipolarer Rotation sowie ionischer Leitfähigkeit stehen und $\omega = 2\pi f$ die Kreisfrequenz ist. Der Beitrag aus der dipolaren Rotation kann bei den bevorzugten Frequenzen (3.5 MHz - 100 MHz) vernachlässigt werden (s. bspw.: Figur 1 in J. Tang, H. Feng, and M. Lau, Microwave heating in food processing, in X. Young and J. Tang, eds., Advances in Bioprocessing Engineering, World Scientific, 2002).

Zusätzlich sind im bevorzugten Frequenzbereich (3.5 MHz bis 100 MHz) niedrige Frequenzen bevorzugt, da diese aus physikalisch-technischen Gründen bei den bevorzugten Elektrodengrössen im Bereich von 1 m² bis 200 m², insbesondere von 4 m² oder 3 m² bis 30 m² und bei einem Generator 13 von grösser oder gleich 200 kW, bei höheren Frequenzen wie z.B. der IMS-Frequenz von 27.12 MHz ($\lambda$ = 1106.2 cm) zu stärkeren Inhomogenitäten im elektrischen Feld H führen, was schlussendlich zur ungleichmässigen Erwärmung (lokalen Kälteinseln und Überhitzungen) des zu behandelten Materials 10 führt. Grosse Elektroden 11, 12 mit Elektrodenflächen 110, 120 bis zu 20 m² sind bei Frequenzen zwischen 3.5 MHz bis 20 MHz wegen der grösseren Wellenlängen ($\lambda$ = 2212.4 cm bei 13.56 MHz) bei einer Generatorleistung von grösser oder gleich 200 kW gut beherrschbar, sie bilden bei geeigneter Steuerung ein weitaus homogeneres Feld H aus. Der bevorzugt hohe Energieeintrag verbunden mit einer hohen Energiedichte (linear ansteigend mit der Frequenz; ca. 0.6 W/cm³) limitiert die möglichen Arbeitsfrequenzen auf Werte im Bereich von 3.5 MHz bis 20 MHz, weshalb diese bevorzugt sind. Besonders bevorzugt ist die ISM-Frequenz von 13.56 MHz, da damit ein energetisch und ökonomisch sinnvolles wie auch anlagentechnisch realisierbares Verfahren gemäss Patentanspruch gewährleistet ist. Verfahren mit Frequenzen ausserhalb der genannten ISM-Werte sind aus rechtlichen Gründen (Funkverkehr) nur abgeschirmt möglich (Faraday'scher Käfig), technisch sehr aufwändig und ökonomisch nachteilig, also weniger bevorzugt.

Besonders vorteilhaft in dieser Anlagenkonstruktion ist das modulare System. Mehrere Behandlungsvorrichtungen 1 können parallel wie auch seriell im Verbund betrieben werden und Förderstrassen 141 bilden, was der Kapazität dieses Verfahrens nach oben hin praktisch keine Grenzen setzt. Die Stundenleistung derartiger Verbundsysteme liegt je nach Anzahl und Dimension der Einheiten 1 bei 10 Tonnen bis 500 Tonnen pro Stunde.

In **Figur 3** zeigt eine bevorzugte Ausführungsform für eine Grossanlage. Ein Verfahren zur grosstechnischen und kontinuierlichen Zerstörung der Keim- und/oder Fortpflanzungsfähigkeit von Beimengungen wie invasiven Neophyten in Behandlungsgut 10 wie Aushubmaterial ist bis dato nicht bekannt.

Gemäss dieser Ausführungsform wird ein grosstechnisches, kontinuierliches Verfahren gelehrt, welches Tonnen von Behandlungsgut 10 pro Stunde mit dem hochfrequente elektrische Felder H im Bereich von 3.5 MHz bis 100 MHz, insbesondere mit einer Frequenz von 13.56 MHz, und mit Elektrodenflächen 110, 120 mit bis zu 200 m² und einem Kondensatorplattenabstand von 10 cm bis 100 cm behandelt. Der Materialstrom kann also eine Schichtdicke h von im Wesentlichen 10 cm bis 100 cm oder mehr aufweisen. Die hierin genannten Elektrodengrössen, Generatorleistungen und Feldparameter erlauben es, den so dimensionierten Materialstrom nachhaltig und zuverlässig über den gesamten Querschnitt zu behandeln. Es kann somit eine grosstechnische Anlage mit einer Verarbeitungsleistung von mehreren hundert Tonnen Behandlungsmaterial pro Stunde bereitgestellt werden.

Eine Anlage im Tonnenmassstab zur grosstechnischen und kontinuierlichen Zerstörung der Keim- und/oder Fortpflanzungsfähigkeit von invasiven Neophyten und damit belastetem Aushubmaterial ist in **Fig. 3** dargestellt. Mit Neophyten belastetes Aushubmaterial fällt allgemein mit einer Feuchtigkeit von 5 % bis 40 % an. Ein grosstechnisches Verfahren mit einer Behandlungsleistung von 10 t/h bis 500 t/h benötigt 0.1 MW bis 10 MW an Leistung um die erforderliche Temperatur der aktiven Beimengungen 100 auf ein $T_{max}$ von 50 °C bis 80 °C zu erreichen.

Grundsätzlich wird das im Zusammenhang mit **Fig. 2** beschriebene Verfahren angewendet, wobei bei der Anlage gemäss **Fig. 3** mehrere parallele Förderstrassen 141 genutzt werden. Diese Förderstrassen 141 können jeweils aus mehreren sich aneinander anschliessenden Förderbändern 140 gebildet sein, wobei jeder Förderstrasse 141 eine oder mehrere Generatoreinheiten 13 und Elektrodenanordnungen 11, 12 zugeordnet sein können.

Ein weiterer Vorteil besteht im flexiblen Einsatz der Anlagenkonstruktion. Je nach Notwendigkeit werden diese Einheiten stationär in Aufbereitungsanlagen oder z. B. Kiesgruben eingesetzt. Transportabel auf einem entsprechend ausgerüsteten Fahrzeug montiert sind sie direkt am mit Neophyten belastetem Standort einsetzbar.

Beim erfindungsgemässen Verfahren wird also belastetes Behandlungsgut 10 zur thermischen Eliminierung der Keim- und/oder Fortpflanzungsfähigkeit von keim- und/oder fortpflanzungsfähigen Beimengungen 100 im Behandlungsgut 10 angeliefert, vorzugsweise mit einem Transportmittel 20. Das Gut 10 wird der Aufgabestation 4 des Vorrichtung 1 übergeben.

Mittels der Generatoreinheit 13 wird ein Radiofrequenzsignal S erzeugt, vorzugsweise mit einer Frequenz f von 3.5 MHz bis 100 MHz, insbesondere von 10 MHz bis 20 MHz, besonders bevorzugt von 13.56 MHz. Dieses hochfrequente Signal S wird auf die Elektrodenanordnung 11, 12 gekoppelt, sodass ein Hochfrequenzfeld H erzeugt wird.

Sodann wird das zu behandelnde Behandlungsgut 102 dem Hochfrequenzfeld H ausgesetzt. Vorzugsweise wird hierzu ein Förderband 140 genutzt, auf welchem das Prozessgut in einer Schichtdicke h von bis zu 100 cm geschichtet ist. Ein solches Förderband kann bspw. 1 m breit sein. Es können auch eine Vielzahl von parallelen Förderstrassen 141 genutzt werden, um Behandlungsgut 10 mit einer Leistung von 10 t/h bis 500 t/h, insbesondere von 50 t/h bis 200 t/h.

Die Verweildauer des Guts 10 pro Volumeneinheit 103, vorzugsweise 10 Sekunden bis 600 Sekunden oder länger, die Generatorleistung, vorzugsweise 20 kW bis 500 kW pro Elektrodenpaar, und die Grösse der Elektrodenflächen 110, 120, vorzugsweise von 1 m$^2$ bis 20 m$^2$ werden nun so gewählt, dass die Beimengungen 100 auf eine maximale Temperatur $T_{max}$ von 50 °C bis 80 °C, vorzugsweise auf eine Temperatur von 65 °C bis 75 °C erwärmt werden. Ist die Temperatur $T_{max}$ höher, so leidet die Energieeffizienz, ist sie kleiner, so wird der Inaktivierungsprozess nicht zuverlässig genug ausgeführt.

Die im behandelten Gut 10 verbleibende Prozesswärme wird vorzugweise teilweise zurückgewonnen und dem zu behandelnden Prozessgut zwecks Vorwärmung zugeführt.

Während des Behandlungsprozesses kann das Transportmittel gewaschen werden.

BEZUGSZEICHENLISTE

| | | | | |
|---|---|---|---|---|
| 1 | Behandlungsvorrichtung | | | |
| 2 | Gehäuse | | 14 | Fördervorrichtung |
| 21 | erste Kammer | | 140 | Förderband |
| 22 | zweite Kammer | | 141 | Förderstrasse |
| 23 | dritte Kammer | | | |
| | | | 15 | Waschvorrichtung |
| 3 | Isolation | | | |
| 4 | Aufgabestation | | 16 | Wärmerückgewinnungsvorrichtung |
| 5 | Behandlungsgutabgabe | | | |
| | | | 17 | Bezahlvorrichtung |
| 10 | Behandlungsgut | | | |
| 100 | aktive Beimengungen in 10 | | 20 | Transportmittel |
| 101 | behandeltes Behandlungsgut | | | |
| 102 | zur Behandlung vorgesehenes Behandlungsgut | | 31 | Transformator |
| | | | 32 | Gleichrichter |
| 103 | Volumenelement von 10 | | 33 | Leistungstriode |
| 105 | Austauschfluid | | 34 | Koppelspule |
| 106 | inaktivierte Beimengung | | 35 | Anpassvorrichtung |
| | | | 36 | Arbeitskondensator |
| 11 | erste Elektrode der Elektrodenanordnung | | ET | Eindringtiefe von H in 10 |
| 110 | erste Elektrodenfläche von 11 | | f | Frequenz von H |
| 12 | zweite Elektrode der Elektrodenanordnung | | h | Materialhöhe von 10 |
| | | | H | elektrisches Hochfrequenzfeld |
| 120 | zweite Elektrodenfläche von 12 | | S | Radiofrequenzsignal |
| | | | $T_{max}$ | maximale Temperatur von 10 |
| 13 | Generatoreinheit | | T, R, O, A,C | Elektrische Stufen |
| 130 | Kühlung | | | |

**EP 3 295 791 B1**

**Patentansprüche**

1. Verfahren zur thermischen Eliminierung der Keim- und/oder Fortpflanzungsfähigkeit von keim- und/oder fortpflanzungsfähigen pflanzlichen Beimengungen (100) in Behandlungsgut (10) in Form von Aushubmaterial, umfassend folgende Schritte:

   a) Erzeugen eines Radiofrequenzsignals (S) mittels einer Generatoreinheit (13);
   b) Erzeugung eines elektrischen Hochfrequenzfeldes (H) mit dem Radiofrequenzsignal (S) mittels einer Elektrodenanordnung (11,12);
   c) Bestrahlung des Behandlungsgutes (10) mit dem elektrischen Hochfrequenzfeld (H), sodass gezielt die keim- und/oder fortpflanzungsfähigen pflanzlichen Beimengungen (100) auf eine Temperatur von 50 °C bis 80 °C, vorzugsweise von 65 °C bis 75 °C erwärmt werden, wobei das elektrische Hochfrequenzfeld (H) eine Frequenz (f) von 3.5 MHz bis 100 MHz, insbesondere von 10 MHz bis 20 MHz, besonders bevorzugt von 13.56 MHz aufweist, und wobei das Behandlungsgut (10) eine Dichte zwischen 1.0 g/cm$^3$ bis 3.0 g/cm$^3$ und einen Wassergehalt von 5 % bis 40 % aufweist, sodass sich das restliche Material des Behandlungsgutes (10) weniger stark aufheizt als die keim- und/oder fortpflanzungsfähigen pflanzlichen Beimengungen (100).

2. Verfahren nach Anspruch 1, wobei eine Verweildauer des Behandlungsguts (10) im Hochfrequenzfeld (H) jeweils 10 Sekunden bis 600 Sekunden, insbesondere 120 Sekunden bis 300 Sekunden pro Volumenelement (103) beträgt.

3. Verfahren nach einem der Ansprüche 1 bis 2, wobei das Behandlungsgut (10) geschichtet mit einer Materialhöhe (h) von 10 cm bis 100 cm, vorzugsweise von 20 cm bis 50 cm dem elektrischen Hochfrequenzfeld (H) ausgesetzt wird.

4. Verfahren nach einem der Ansprüche 1 bis 3, wobei das Behandlungsgut (10) kontinuierlich durch das elektrische Hochfrequenzfeld (H) geführt wird.

5. Verfahren nach einem der Ansprüche 1 bis 4, wobei Behandlungsgut (10) in der Menge von 10 t/h bis 500 t/h, insbesondere von 50 t/h bis 200 t/h in einer Grossanlage mit einer Fördervorrichtung (14) behandelt wird.

6. Verfahren nach einem der Ansprüche 1 bis 5, wobei die mindestens einen ersten und/oder zweiten Elektroden (11,12) als mobile Elektroden, vorzugsweise als Bodenelektroden, Sonden, Lanzen oder Pflugscharen zum, in oder durch das Behandlungsgut geführt werden.

7. Behandlungsvorrichtung (1) zur Behandlung von Behandlungsgut (10) in Form von Aushubmaterial, wobei das Behandlungsgut (10) keim- und/oder fortpflanzungsfähige pflanzliche Beimengungen (100) aufweist und die Behandlung die thermischen Eliminierung der Keim- und/oder Fortpflanzungsfähigkeit der keim- und/oder fortpflanzungsfähigen Beimengungen umfasst, wobei die Behandlungsvorrichtung (1) umfasst:

   mindestens eine Generatoreinheit (13) zur Bereitstellung eines Radiofrequenzsignals (S);
   mindestens eine Elektrodenanordnung (11,12) umfassend mindestens eine erste Elektrode (11) und mindestens eine zweite Elektrode (12) zur Bereitstellung eines elektrischen Hochfrequenzfeldes (H) aufgrund des Radiofrequenzsignales (S);
   **dadurch gekennzeichnet, dass** die Behandlungsvorrichtung (1) eingerichtet ist, gezielt die keim- und/oder fortpflanzungsfähigen pflanzlichen Beimengungen (100) auf eine Temperatur von 50 °C bis 80 °C, vorzugsweise von 65 °C bis 75 °C zu erwärmen, wobei das elektrische Hochfrequenzfeld (H) eine Frequenz (f) von 3.5 MHz bis 100 MHz, insbesondere von 10 MHz bis 20 MHz, besonders bevorzugt von 13.56 MHz aufweist, und wobei das Behandlungsgut (10) eine Dichte zwischen 1.0 g/cm$^3$ bis 3.0 g/cm$^3$ und einen Wassergehalt von 5 % bis 40 % aufweist, sodass sich das restliche Material des Behandlungsgutes (10) weniger stark aufheizt als die keim- und/oder fortpflanzungsfähigen pflanzlichen Beimengungen (100), und wobei die mindestens einen ersten und zweiten Elektroden (11,12) als mobile Elektroden in Form von Pflugscharen ausgebildet sind und geeignet sind, durch das Behandlungsgut geführt zu werden.

8. Behandlungsvorrichtung (1) nach Anspruch 7, umfassend mindestens eine Fördervorrichtung (14) zur Beförderung von Behandlungsgut (10) durch das elektrische Hochfrequenzfeld (H).

9. Behandlungsvorrichtung (1) nach einem der Ansprüche 7 bis 8, welche weiter eingerichtet ist, eine Verweildauer des Behandlungsguts (10) im elektrischen Hochfrequenzfeld (H) von 10 Sekunden bis 600 Sekunden, insbesondere von 120 Sekunden bis 300 Sekunden im elektrischen Hochfrequenzfeld (H) zu realisieren.

**10.** Behandlungsvorrichtung (1) nach einem der Ansprüche 7 bis 9, wobei eine Wärmerückgewinnungsvorrichtung (16) vorgesehen ist, welche eingerichtet ist,

a) behandeltes Behandlungsgut (101) und/oder die Generatoreinheit (13) mit einem Austauschfluid (105) in Wärmekontakt zu bringen, sodass das Austauschfluid Wärme aufnimmt, und danach
b) das Austauschfluid (105) mit der aufgenommenen Wärme mit zur Behandlung vorgesehenem Behandlungsgut (102) zwecks Vorwärmung in Kontakt zu bringen.

**11.** Behandlungsvorrichtung (1) nach Anspruch 7 bis 10, wobei die Fördervorrichtung (14) ausgebildet ist, das Behandlungsgut (10) kontinuierlich durch das elektrische Hochfrequenzfeld (H) zu führen, wobei die Fördervorrichtung (14) vorzugsweise ein einzelnes oder eine Vielzahl von Förderbändern (140) umfasst.

**12.** Behandlungsvorrichtung (1) nach Anspruch 11, wobei die Fördervorrichtung (14) eine Vielzahl von parallelen Förderstrassen (141) aufweist, wobei jede Förderstrasse (141) vorzugsweise mindestens eine Elektrodenanordnung (11,12) aufweist, wobei die Behandlungsvorrichtung (10) vorzugsweise ausgelegt ist, um Behandlungsgut (10) in der Menge von 10 t/h bis 500 t/h, insbesondere von 50 t/h bis 200 t/h zu behandeln.

**13.** Behandlungsvorrichtung (1) nach einem der Ansprüche 7 bis 12,
wobei die Generatoreinheit (13) eine Ausgangsleistung 20 kW bis 3000 kW aufweist und/oder wobei eine Ausgangsspannung der Generatoreinheit (13) 0.5 kV bis 15 kV beträgt.

**14.** Behandlungsvorrichtung (1) nach einem der Ansprüche 7 bis 13, wobei eine Elektrodenfläche (110,120) der ersten und/oder zweiten Elektrode (11;12) im Bereich zwischen 1 m$^2$ und 200 m$^2$, insbesondere zwischen 3 m$^2$ und 30 m$^2$, liegt, wobei ein Abstand zwischen den beiden Elektrodenflächen (110, 120) 10 cm bis 100 cm, insbesondere 20 cm bis 50 cm beträgt.

**15.** Behandlungsvorrichtung (1) nach einem der Ansprüche 7 bis 14 weiter umfassend eine Waschvorrichtung (15) zur Reinigung der Transportmittel (20), insbesondere Lastwagen, während der Behandlung von Behandlungsgut (10).

**16.** Verwendung des Verfahrens nach einem der Ansprüche 1 bis 6 und/oder der Behandlungsvorrichtung nach einem der Ansprüche 7 bis 15 zur Behandlung von Behandlungsgut (10) mit pflanzlichen Beimengungen (100), wobei diese pflanzlichen Beimengungen (100) Neophyten umfassen oder Neophyten sind.

## Claims

**1.** Method for the thermal elimination of the germination capacity and/or fertility of plant constituents capable of germination and/or reproduction (100) in material to be treated (10) in the form of excavation material, comprising the following steps:

a) producing a radio frequency signal (S) by a generator unit (13);
b) producing an electric high-frequency field (H) with the radio frequency signal (S) by means of an electrode arrangement (11, 12);
c) irradiation of the material to be treated (10) with the electric high-frequency field (H), such that specifically the plant constituents capable of germination and/or reproduction (100) are heated to a temperature of 50°C to 80°C, preferably of 65°C to 75°C, wherein the electric high-frequency field (H) has a frequency (f) of 3.5 MHz to 100 MHz, preferably of 10 to 20 MHz, more preferably of 13.56 MHz, and wherein the material to be treated (10) has a density of between 1.0 g/cm$^3$ to 3.0 g/cm$^3$ and a water content of 5% to 40%, such that the rest of the material of the material to be treated (10) heats up less than the plant constituents capable of germination and/or reproduction (100).

**2.** Method according to claim 1, wherein an exposure time of the material to be treated (10) in the high-frequency field (H) is in each case 10 seconds to 600 seconds, preferably 120 seconds to 300 seconds per volume element (103).

**3.** Method according to one of claims 1 to 2, wherein the material to be treated (10) is exposed to the electric high-frequency field (H) in a stacked manner with a material height (h) of 10 cm to 100 cm, preferably of 20 cm to 50 cm.

**4.** Method according to one of claims 1 to 3, wherein the material to be treated (10) is guided through the electric high-frequency field (H) in a continuous manner.

5. Method according to one of claims 1 to 4, wherein the material to be treated (10) is treated in an amount of 10 t/h to 500 t/h, preferably of 50 t/h to 200 t/h in a large-scale plant with a conveyer device (14).

6. Method according to one of claims 1 to 5, wherein the at least one first and/or second electrodes (11, 12) are guided as mobile electrodes, preferably as ground electrodes, probes, lances, or plough shares to, in or through the material to be treated.

7. Treatment device (1) for the treatment of material to be treated (10) in the form of excavation material, wherein the material to be treated (10) comprises plant constituents capable of germination and/or reproduction (100) and wherein the treatment comprises the thermal elimination of the germination capacity and/or fertility of the constituents capable of germination and/or reproduction (100), wherein the treatment device (1) comprises:

   - at least one generator unit (13) for the production of a radio frequency signal (S);
   - at least one electrode arrangement (11, 12), comprising at least one first electrode (11) and at least one second electrode (12) for the production of an electric high-frequency field (H) based on the radio frequency signal (S);

   **characterized in that** the treatment device (1) is equipped to specifically heat the plant constituents capable of germination and/or reproduction (100) to a temperature of 50°C to 80°C, preferably of 65°C to 75°C, wherein the electric high-frequency field (H) comprises a frequency (f) of 3.5 MHz to 100 MHz, preferably of 10 MHz to 20 MHz, more preferably of 13.56 MHz, and wherein the material to be treated (10) comprises a density between 1.0 g/cm$^3$ to 3.0 g/cm$^3$ and a water content of 5% to 40%, such that the rest of the material of the material to be treated (10) heats up less than the plant constituents capable of germination and/or reproduction (100), and wherein the at least one first and second electrodes (11, 12) are formed as mobile electrodes in the form of plough shares and are suitable to be guided through the material to be treated.

8. Treatment device (1) according to claim 7, comprising at least one conveyer device (14) for conveying material to be treated (10) through the electric high-frequency field (H).

9. Treatment device (1) according to one of claims 7 to 8, which is further equipped to realize an exposure time of the material to be treated (10) in the electric high-frequency field (H) of 10 seconds to 600 seconds, preferably of 120 seconds to 300 seconds.

10. Treatment device (1) according to one of claims 7 to 9, wherein a heat recovery device (16) is provided, which is equipped to

   a) bring treated material (101) and/or the generator unit (13) in heat contact with an exchange fluid (105), such that the exchange fluid absorbs heat, and subsequently
   b) bring the exchange fluid (105) with the absorbed heat in contact with the material to be treated which is designated for treatment (102), for the purpose of pre-heating.

11. Treatment device (1) according to one of claims 7 to 10, wherein the conveyer device (14) is equipped to guide the material to be treated (10) through the electric high-frequency field (H) in a continuous manner, wherein the conveyer device (14) preferably comprises a single or a multitude of conveyer belts (140).

12. Treatment device (1) according to claim 11, wherein the conveyer device (14) comprises a multitude of parallel conveyer lanes (141), wherein each conveyer lane (141) preferably comprises at least one electrode arrangement (11, 12), wherein the treatment device (10) preferably is equipped to treat material to be treated (10) in the amount of 10 t/h to 500 t/h, preferably of 50 t/h to 200 t/h.

13. Treatment device (1) according to one of claims 7 to 12, wherein the generator unit (13) comprises an output power of 20 kW to 3000 kW, and/or wherein an output voltage of the generator unit (13) is 0.5 kV to 15 kV.

14. Treatment device (1) according to one of claims 7 to 13, wherein an electrode surface (110, 120) of the first and/or the second electrode (11, 12) lies in the range of between 1 m$^2$ and 200 m$^2$, preferably of 3 m$^2$ to 30 m$^2$, wherein a distance between the two electrode surfaces (110, 120) is 10 cm to 100 cm, preferably 20 cm to 50 cm.

**15.** Treatment device (1) according to one of claims 7 to 14, further comprising a washing device (15) for cleaning the transport means (20), preferably trucks, during the treatment of material to be treated (10).

**16.** Use of the method according to one of claims 1 to 6 and/or of the treatment device according to one of claims 7 to 15 for the treatment of material to be treated (10) comprising plant constituents (100), wherein these plant constituents (100) comprise neophytes or are neophytes.

**Revendications**

**1.** Un procédé d'élimination thermique de la capacité de germination et/ou reproduction d'ajouts végétaux capables de germiner et/ou de se reproduire (100) dans un matériau à traiter (10) en forme de matériau excavé, comportant les étapes suivantes:

   a) générer un signal de fréquence radio (S) au moyen d'une unité génératrice (13);
   b) générer un champ électrique à haute fréquence (H) à l'aide du signal de fréquence radio (S) au moyen d'un agencement d'électrodes (11,12);
   c) irradiation matériau à traiter (10) avec le champ électrique à haute fréquence (H), de sorte à ce que les ajouts végétaux (100) capables de germiner et/ou de se reproduire sont chauffés à une température de 50°C à 80°C de manière ciblée, préférablement de 65°C à 75°C, où le champ électrique à haute fréquence (H) présente une fréquence (f) de 3.5 MHz à 100 MHz en particulier de 10 MHz à 20 MHz, en particulier préférablement de 13.56 MHz, et où le matériau à traiter (10) présente une densité entre 1.0 g/cm$^3$ à 3.0 g/cm$^3$ et un contenu d'eau de 5% à 40%, de sorte que le matériau restant du matériau à traiter (10) est chauffé de manière moins importante que les ajouts végétaux capables de germiner et/ou de se reproduire (100) .

**2.** Un procédé selon la revendication 1, où la durée de résidence du matériau à traiter (10) dans le champ électrique à haute fréquence (H) correspond respectivement à 10 secondes à 600 secondes, en particulier de 120 secondes à 300 secondes par élément de volume (103).

**3.** Un procédé selon une des revendications 1 à 2, où le matériau de traitement (10) est exposé au champ électrique à haute fréquence (H) sous forme stratifiée ayant une hauteur de matériau (h) de 10 cm à 100 cm, préférablement de 20 cm à 50 cm.

**4.** Un procédé selon une des revendications 1 à 3, où le matériau à traiter (10) est guidé de manière continue à travers le champ électrique à haute fréquence (H).

**5.** Un procédé selon une des revendications 1 à 4, où le matériau de traitement (10) est traité en quantité de 10 t/h à 500 t/h, en particulier 50 t/h à 200 t/h dans une installation munie d'un dispositif de convoyage (14).

**6.** Un procédé selon une des revendications 1 à 5, où au moins une de la première et/ou de la deuxième électrode (11, 12) est guidée vers, dans ou à travers, le matériau à traiter (10) en tant qu'électrodes mobiles, préférablement en tant qu'électrodes de sol, sondes, lances ou charrues.

**7.** Un dispositif de traitement (1) pour le traitement de matériau à traiter (10) en forme de matériau excavé, dans lequel le matériau à traiter (10) présente des ajouts végétaux capables de germiner et/ou de se reproduire (100) et le traitement comprend l'élimination thermique de la capacité de germiner et/ou de se reproduire des d'ajouts végétaux capables de germiner et/ou de se reproduire (100), où le dispositif de traitement (1) comprend:

   au moins une unité génératrice (13) pour mettre à disposition un signal de fréquence radio (S) ;
   au moins un agencement d'électrodes (11,12) comprenant au moins une première électrode (11) et au moins une seconde électrode (12) pour mettre à disposition un champ électrique à haute fréquence (H) à l'aide du signal de fréquence radio (S);
   **caractérisé en ce que**
   le dispositif de traitement (1) est agencé pour chauffer les ajouts végétaux (100) capables de germiner et/ou de se reproduire à une température de 50°C à 80°C de manière ciblée, préférablement de 65°C à 75°C, où le champ électrique à haute fréquence (H) présente une fréquence (f) de 3.5 MHz à 100 MHz en particulier de 10 MHz à 20 MHz, en particulier préférablement de 13.56 MHz, et où le matériau à traiter (10) présente une densité entre 1.0 g/cm3 à 3.0 g/cm3 et un contenu d'eau de 5% à 40%, de sorte que le matériau restant du matériau à traiter (10) est chauffé de manière moins importante que les ajouts végétaux capables de germiner et/ou de se reproduire (100), et

où l'au moins une première électrode (11) et l'au moins une deuxième électrode (12) sont conçues en forme d'électrodes mobiles en tant que charrues et sont susceptibles d'être guidées à travers le matériau à traiter (10).

8. Un dispositif de traitement (1) selon la revendication 7, comprenant un dispositif de convoyage (14) pour convoyer le matériau de traitement (10) à travers le champ électrique à haute fréquence (H).

9. Un dispositif de traitement (1) selon une des revendications 7 à 8, lequel est agencé pour réaliser une durée de résidence du matériau à traiter (10) dans le champ électrique à haute fréquence (H) de 10 secondes à 600 secondes, en particulier de 120 secondes à 300 secondes.

10. Un dispositif de traitement (1) selon une des revendications 7 à 9, où un dispositif de récupération de chaleur (16) est prévu, lequel est agencé pour,

   a. mettre en contact thermique un matériau ayant été soumis à un traitement (101) et/ou l'unité génératrice (13) avec un fluide d'échange (105) de sorte à ce que le fluide d'échange (105) absorbe de la chaleur;
   b. mettre en contact le fluide d'échange (105) ayant absorbé de la chaleur avec le matériau destiné à être soumis à un traitement (102) afin de préchauffer celui-ci.

11. Un dispositif de traitement (1) selon une des revendications 7 à 10, dans lequel le dispositif de convoyage (14) est agencé pour guider le matériau à traiter (10) de manière continue à travers le champ électrique à haute fréquence (H), où le dispositif de convoyage (14) comprend préférablement une bande de convoyage individuelle ou une pluralité de bandes de convoyage (140).

12. Un dispositif de traitement (1) selon la revendication 11, dans lequel dans lequel le dispositif de convoyage (14) comprend une pluralité de bandes de convoyage parallèles (141), chaque bande de convoyage (141) comportant de préférence au moins un agencement d'électrodes (11, 12), où le dispositif de traitement (10) est de préférence conçu pour traiter le matériau à traiter (10) à raison de 10 t/h à 500 t/h, en particulier de 50 à 200 t/h.

13. Un dispositif de traitement (1) selon une des revendications 7 à 12, dans lequel l'unité génératrice (13) présente une puissance de sortie de 20 kW à 3 000 kW et/ou dans lequel l'unité génératrice (13) présente une tension de sortie comprise entre 0,5 kV et 15 kV.

14. Un dispositif de traitement (1) selon une des revendications 7 à 13, dans lequel une surface d'électrode (110, 120) de la première et/ou de la deuxième électrode (11; 12) se situe entre 1 m$^2$ et 200 m$^2$, en particulier 3 m$^2$ et 30 m$^2$, où la distance entre les deux surfaces d'électrodes (110, 120) étant comprise entre 10 cm et 100 cm, en particulier entre 20 cm et 50 cm.

15. Un dispositif de traitement (1) selon l'une des revendications 7 à 14, comprenant en outre un dispositif de lavage (15) pour nettoyer les moyens de transport (20), en particulier les camions, pendant le traitement du matériau à traiter (10).

16. Utilisation du procédé selon l'une des revendications 1 à 6 et / ou du dispositif de traitement selon l'une des revendications 7 à 15 pour le traitement de matériau à traiter (10) avec des ajouts végétaux (100), ces ajouts végétaux (100) comprenant des néophytes ou étant des néophytes.

FIG. 1

FIG. 2

FIG. 3

FIG. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006022611 A1
- US 20150020447 A1
- CA 1298952
- WO 9962566 A1
- NL 1028075 C2


**In der Beschreibung aufgeführte Nicht-Patentliteratur**

- Permittivity and measurements. **KOMAROV, V. ; WANG, S. ; TANG, J.** Encyclopedia of RF and Microwave Engineering. John Wiley and Sons, Inc, 2005, 3693-3711
- *Chemie Ingenieur Technik,* Dezember 1965, vol. 37 (12), 1229-1234
- **A. VON HIPPEL.** Dielectrics and Waves. Wiley, 1954
- Microwave heating in food processing. **J. TANG ; H. FENG ; M. LAU.** Advances in Bioprocessing Engineering, World Scientific. 2002
- Microwave heating in food processing. **J. TANG ; H. FENG ; M. LAU.** Advances in Bioprocessing Engineering. World Scientific, 2002